(19) Europäisches Patentamt
European Patent Office
Office européen des brevets

(11) Veröffentlichungsnummer: **0 218 171 A2**

(12) **EUROPÄISCHE PATENTANMELDUNG**

(21) Anmeldenummer: **86113394.0**

(22) Anmeldetag: **29.09.86**

(51) Int. Cl.⁴: **C07D 239/36**, C07D 239/52

(30) Priorität: **09.10.85 DE 3536065**
**18.12.85 DE 3544691**
**15.05.86 DE 3616337**

(43) Veröffentlichungstag der Anmeldung:
**15.04.87 Patentblatt 87/16**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Arndt, Michael, Dr.**
**Cäcilienstrasse 5**
**D-5600 Wuppertal 1(DE)**
Erfinder: **Heinrich, Josef, Dr.**
**Königsbergerstrasse 5**
**D-5650 Solingen 19(DE)**

(54) **Verfahren zur Herstellung von 2-Alkylsulfonylalkylen-pyrimidinen.**

(57) Die vorliegende Erfindung betrifft ein neues Vefahren zur Herstellung von 2-Alkyl-sulfonylalkylen-pyrimidinen der Formel

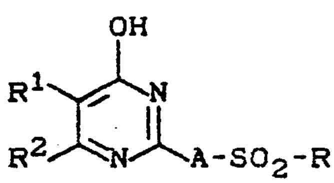

(I)

worin A, R, $R^1$ und $R^2$ die im Anmeldungstext angegebenen Bedeutung besitzen.

Das neue Verfahren ist dadurch gekennzeichnet, daß man zum Erhalt eines Reaktionsgemisches (1)

Acetonitrile $Cl-A-CH_2-CN$, Ammoniumhalogenide $NH_4X$ und Alkylmercaptane RSH in Gegenwart von Säureakzeptoren und von Verdünnungsmitteln bei -20° C bis +60° C umsetzt, weiterhin zum Erhalt des Reaktionsgemisches (2)

Essigsäureester $R^1-CH_2-COOR^3$ und Ester $R^2-COOR^4$ in Gegenwart von Alkoholat oder einer alkoholischen Alkoholatlösung und eines inerten Verdünnungsmittels bei 0 bis 40° C umsetzt,

anschließend die beiden Reaktionsgemische (1) und (2) zusammengibt, einengt und in Gegenwart katalytischer Mengen eines Vanadium-, Wolfram- oder Molybdänsalzes mit Wasserstoffperoxid zu den Sulfonylverbindungen der Formel (I) umsetzt.

Die Verbindungen (I) dienen als Zwischenprodukte für hochwirksame Insektizide.

EP 0 218 171 A2

## Verfahren zur Herstellung von 2-Alkylsulfonylalkylen-pyrimidinen

Die Erfindung betrifft ein neues Verfahren zur Herstellung von 2-Alkylsulfonylalkylen-pyrimidinen.

Es ist bekannt, daß man 2-Alkylsulfonylalkylen-pyrimidine erhält, wenn man Alkylthioalkylenamidine mit β-Ketoestern zu den 2-Alkylthioalkylen-pyrimidinen umsetzt und die 2-Alkylthioalkylen-pyrimidine nach Aufarbeitung und Isolierung zu den entsprechenden Sulfonyl-Derivaten oxidiert (vgl. z.B. DE-OS 3 324 399, DE-OS 3 211 035, DE-OS 2 928 185 und DE-OS 2 838 359).

Die Alkylthioamidine und die β-Ketoester können durch Umsetzung von Acetonitril-Derivaten mit Ammoniumhaloge niden und Alkylmercaptanen bzw. Essigsäureester-Derivaten mit Ester-Derivaten hergestellt und nach Aufarbeitung und Isolierung als Vorprodukte eingesetzt werden.

Die Nachteile bei dieser Herstellungsmethode bestehen darin, daß man die 2-Alkylsulfonylalkylen-pyrimidine über mehrstufige Prozesses erhält, die Ausbeuten dadurch insgesamt unbefriedigend sind und für die Aufarbeitung und Isolierung der einzelnen Vorprodukte ein hoher Energie-und Zeitaufwand erforderlich sind.

Es wurde nun gefunden, daß man 2-Alkylsulfonylalkylenpyrimidine der Formel (I)

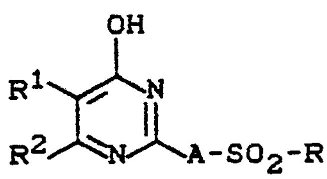

(I)

in welcher

R für geradkettiges oder verzweigtes Alkyl steht,

$R^1$ für Wasserstoff oder für gegebenenfalls substituierte Reste aus der Reihe Alkyl, Alkoxy oder Alkylsulfonyl steht,

$R^2$ für Wasserstoff oder gegebenenfalls substituiertes Alkyl steht und

A für geradkettiges oder verzweigtes Alkylen steht, erhält, wenn man

zum Erhalt eines Reaktionsgemisches (1)

(1) Chloralkylennitrile der Formel (II)

Cl-A-CN (II)

in welcher

A die oben angegebene Bedeutung hat, mit Ammoniumhalogeniden der Formel (III)

$NH_4X$ (III)

in welcher

X für Halogen steht,

und Alkylmercaptanen der Formel (IV)

RSH (IV)

in welcher

R die oben angegebene Bedeutung hat,

in Gegenwart von Säureakzeptoren und in Gegenwart von Verdünnungsmitteln, bei Temperaturen zwischen -20° C und +60° C versetzt; weiterhin

zum Erhalt eines Reaktionsgemisches (2),

(2) Essigsäureester der Formel (V)

$R^1$-$CH_2$-$COOR^3$ (V)

in welcher

$R^1$ die oben angegebene Bedeutung hat und

$R^3$ für Alkyl mit 1-4 C-Atomen steht,

mit Estern der Formel (VI)

$R^2$-$COOR^4$ (VI)

in welcher

$R^2$ die oben angegebene Bedeutung besitzt und

$R^4$ für Alkyl mit 1-4 C-Atomen steht, in Gegenwart von Alkoholat (vorzugsweise Alkalialkoholat) oder einer alkoholischen Alkalialkoholatlösung und in Gegenwart eines inerten Verdünnungsmittels, bei einer Temperatur von 0° C bis 40° C versetzt; und anschließend bei einer Temperatur von 0° C bis 40° C das Reaktionsgemisch (1) zum Reaktionsgemisch (2) gibt, nach Beendigung der Reaktion den entstandenen Alkohol vorzugsweise destillativ entfernt, den Rückstand gegebenenfalls mit Wasser und einer Mineralsäure versetzt, einengt und ohne weitere Reinigung und/oder Isolierung in Gegenwart katalytischer Mengen eines Vanadium-, Wolfram-oder Molybdänsalzes, in Gegenwart von Wasser als Verdünnungsmittel mit Wasserstoffperoxid bei Temperaturen zwischen -20° C und +100° C umsetzt.

Überraschenderweise kann man nach dem erfindungsgemäßen Verfahren die 2-Alkylsulfonylalkylen-pyrimidine der allgemeinen Formel (I) ohne Isolierung von Zwischenstufen in guten Ausbeuten und in hoher Reinheit erhalten, wo doch zu erwarten war, daß eine Aneinanderreihung der obigen Reaktionsschritte ohne Isolierung und Reinigung der Zwischenprodukte nicht zu den gewünschten Produkten oder wegen Nebenreaktionen in einzelnen Stufen nur zu geringen Ausbeuten an verunreinigten Verbindungen führen würde.

Bevorzugt werden mit dem erfindungsgemäßen Verfahren folgende 2-Alkylsulfonylalkylen-pyrimidine der Formel (I) hergestellt

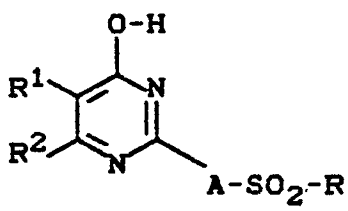

(I)

in welcher

R für geradkettiges oder verzweigtes Alkyl mit 1 bis 6 Kohlenstoffatomen, insbesondere 1 bis 4 Kohlenstoffatomen, steht,

$R^1$ für Wasserstoff oder für Alkyl, Alkoxy, oder Alkylsulfonyl, jeweils mit 1 bis 6 Kohlenstoffatomen, insbesondere 1 bis 4 Kohlenstoffatomen, die gegebenenfalls durch Halogen, $C_1$-$C_2$-Alkoxy oder $C_1$-$C_2$-Alkylsulfonyl substituiert sein können, steht,

$R^2$ für Wasserstoff oder Alkyl mit 1 bis 6 Kohlenstoffatomen, insbesondere 1 bis 4 Kohlenstoffatomen, das gegebenenfalls durch Halogen, insbesondere Chlor oder Fluor, $C_1$-$C_2$-Alkoxy oder $C_1$-$C_2$-Alkylsulfonyl substituiert sein kann, steht und

A für geradkettiges oder verzweigtes Alkylen mit 1 bis 6 Kohlenstoffatomen, insbesondere 1 bis 4 kohlenstoffatomen, steht.

Verwendet man Chloracetonitril, Ammoniumchlorid und Methylmercaptan als Ausgangsstoffe für Reaktion (1); Methoxyessigsäuremethylester, Ameisensäuremethylester und eine methanolische Natriummethanolat-Lösung als Aus gangsstoffe für Reaktion (2) und Wasserstoffperoxid-in Gegenwart von katalytischen Mengen Ammoniummolybdat und Wasser als Oxidationsmittel, so kann die Reaktion des erfindungsgemäßen Verfahrens durch das folgende Formelschema skizziert werden:

(1)

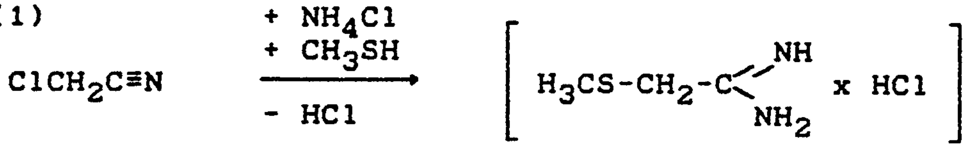


(A)

(2)

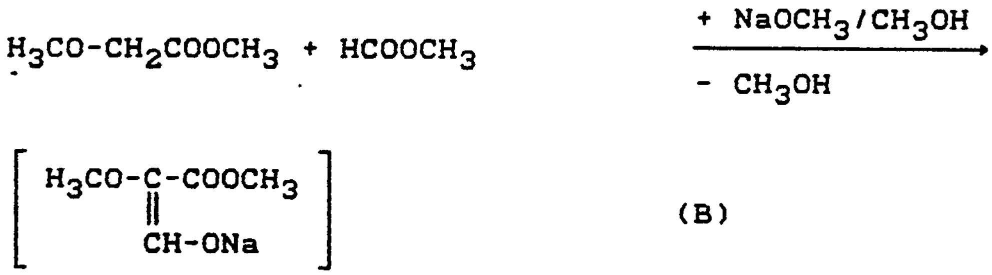


(B)

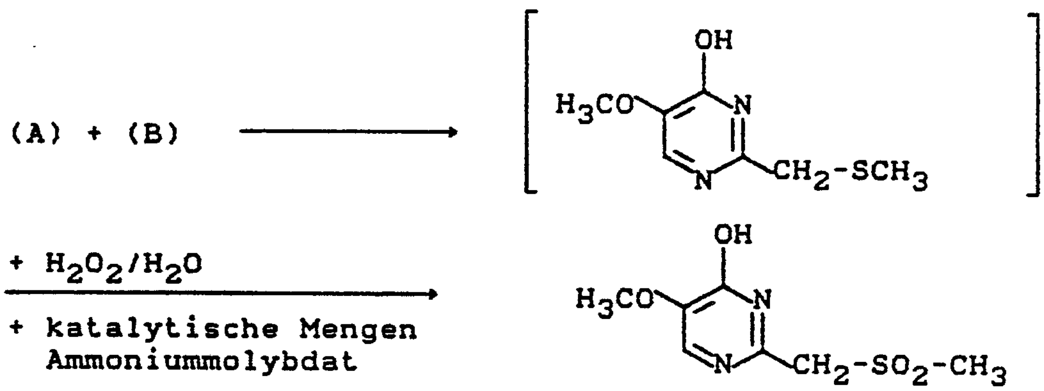


Die Verbindungen, die als Ausgangsstoffe für die Durchführung des erfindungsgemäßen Verfahrens benötigt werden, sind durch die Formel (II) allgemein definiert.

In dieser Formel steht A für diejenigen Reste, welche oben bei der Definition der Verbindungen der Formel (I) genannt sind.

Als Beispiele für die Verbindungen der Formel (II) seien genannt:

Chloracetonitril, 3-Chlorpropionsäurenitril, 2-Chlorpropionsäurenitril, 4-Chlorbuttersäurenitril, 2-Chlorbuttersäurenitril und 5-Chlor-valeriansäurenitril.

Die Verbindungen der Formel (II) sind allgemein bekannte Verbindungen der organischen Chemie.

Die weiterhin als Ausgangsstoffe für das erfindungsgemäße Verfahren benötigten Verbindungen sind durch die Formeln (III), (IV), (V) und (VI) allgemein definiert. In diesen Formeln stehen X, R, $R^1$ und $R^2$ für diejenigen Reste, welche oben bei der Definition der Verbindungen der Formel (I) genannt sind. $R^3$ und $R^4$ stehen bevorzugt für $C_1$-$C_2$-Alkyl.

Als Beispiele für die Verbindungen der Formel (III) seien genannt:

Ammoniumchlorid, -bromid und -iodid.

Als Beispiele für die Verbindungen der Formel (IV) seien genannt:

Methyl-, Ethyl-, n-Propyl-, i-Propyl, n-Butyl-, i-Butyl-, sec.-Butyl-und tert.-Butylmercaptan.

Als Beispiele für die Verbindungen der Formel (V) seien genannt:

$R^1$-$CH_2$-$COOR^3$ (V)

Tabelle 1

| $R^1$ | $R^3$ | $R^1$ | $R^3$ |
|---|---|---|---|
| H | $CH_3$ | H | $C_2H_5$ |
| $CH_3$ | $CH_3$ | $CH_3$ | $C_2H_5$ |
| $OCH_3$ | $CH_3$ | $OCH_3$ | $C_2H_5$ |
| $C_2H_5$ | $CH_3$ | $C_2H_5$ | $C_2H_5$ |
| $OC_2H_5$ | $CH_3$ | $OC_2H_5$ | $C_2H_5$ |
| $C_3H_7$ | $CH_3$ | $C_3H_7$ | $C_2H_5$ |
| $OC_3H_7$ | $CH_3$ | $OC_3H_7$ | $C_2H_5$ |
| $C_3H_7$-i | $CH_3$ | $C_3H_7$-i | $C_2H_5$ |
| $OC_3H_7$-i | $CH_3$ | $OC_3H_7$-i | $C_2H_5$ |
| $SCH_3$ | $CH_3$ | $SCH_3$ | $C_2H_5$ |
| $SC_2H_5$ | $CH_3$ | $SC_2H_5$ | $C_2H_5$ |

Als Beispiele für die Verbindungen der Formel (VI) seien genannt :

Ameisensäuremethylester, Ameisensäureethylester, Essigsäuremethylester und Essigsäureethylester.

Die Verbindungen der Formeln (III), (IV), (V) und (VI) sind allgemein bekannte Verbindungen der organischen Chemie.

Für die Herstellung des Reaktionsgemisches -(1) werden Verdünnungsmittel verwendet. Als solche kommen unter den Reaktionsbedingungen inerte Lösungsmittel in Frage. Hierzu gehören insbesondere Alkohole wie Methanol, Ethanol und n-Propanol. Bevorzugt wird Methanol als Lösungsmittel eingesetzt.

Für die Herstellung des Reaktionsgemisches -(1) werden außerdem Säureakzeptoren verwendet. Als Säureakzeptoren kommen vorzugsweise Alkalialkoholate in Betracht. Bevorzugt werden Natriummethylat oder -ethylat als Säureakzeptor eingesetzt. Als Verdünnungsmittel wird beim Einsetzen von Natriummethylat Methanol, und beim Einsatz von Natriumethylat Ethanol eingesetzt.

Die Herstellung des Reaktionsgemisches (1) erfolgt im allgemeinen bei Temperaturen zwischen -20 ° C und +30 ° C vorzugsweise zwischen -10 ° C und +20 ° C. Die Herstellung des Reaktionsgemisches (1) erfolgt vorzugsweise in einer Inertgasatmosphäre. Als Inertgas kommt vorzugsweise Argon oder Stickstoff in Betracht. Bevorzugt wird in Gegenwart von Stickstoff gearbeitet. Das Reaktionsgemisch (1) wird bevorzugt unter Normaldruck hergestellt.

Bei der Herstellung des Reaktionsgemisches -(1) wird bevorzugt auf 1 Mol Nitril der Formel (II) 1,01 bis 1,25 Mol, vorzugsweise 1,05 bis 1,20 Mol Säureakzeptor, 1 bis 2,00 vorzugsweise 1 bis 1,5 Mol Ammoniumhalogenid der Formel (III) und 1 bis 1,25 Mol, vorzugsweise 1 bis 1,10 Mol Alkylmercaptan der Formel (IV) eingesetzt.

Für die Herstellung des Reaktionsgemisches -(2) werden inerte Verdünnungsmittel verwendet. Hierzu gehören insbesondere aromatische Kohlenwasserstoffe wie Benzol, Xylol, Toluol und Ether, wie Methyl-tert.-butylether, Diethyl-und Dibutylether. Besonders bevorzugt wird Xylol als Verdünnungsmittel eingesetzt.

Für die Herstellung des Reaktionsgemisches -(2) wird entweder festes Alkoholat und/oder eine alkoholische Alkoholatlösung verwendet. Besonders bewährt haben sich Alkalialkoholate, wie Natriummethylat und-ethylat. Wird eine alkohlische Alkoholatlösung verwendet, so wird als Alkohol beim Einsetzen von Natriummethylat, Methanol und beim Einsatz von Natriumethylat Ethanol eingesetzt.

Die Herstellung des Reaktionsgemisches (2) erfolgt im allgemeinen bei Temperaturen zwischen 0 ° C und 40 ° C. Bevorzugt wird der Bereich zwischen 0 ° C und 20 ° C. Das Reaktionsgemisch -(2) wird bevorzugt unter Normaldruck hergestellt.

Bei der Herstellung des Reaktionsgemisches - (2) setzt man auf 1 Mol der Verbindung der Formel (V) 1 bis 3 Mol, vorzugsweise 1,0 bis 2,2 Mol Ester der Formel (VI) und 1 bis 5 Mol, vorzugsweise 1,3 bis 4,2 Mol Alkalialkoholat ein. Die alkoholische Alkalialkoholatlösung enthält auf 1 Mol Alkalialkoholat 0,5 bis 2,5 Mol, vorzugsweise 0,8 bis 2 Mol Alkohol.

Als Mineralsäuren werden für das erfindungsgemäße Verfahren bevorzugt Säuren, wie z.B. Salzsäure, Schwefelsäure, Phosphorsäure und Essigsäure eingesetzt.

Als Katalysatoren für das erfindungsgemäße Verfahren kommen bevorzugt Molybdän-Salze, wie z.B. Ammoniummolybdat, in Betracht.

Das erfindungsgemäße Verfahren wird im allgemeinen bei Temperaturen zwischen -20 ° C und +100 ° C durchgeführt, bevorzugt ist der Temperaturbereich zwischen 0 ° C und 80 ° C, insbesondere zwischen 20 ° C und 60 ° C. Die Umsetzungen werden im allgemeinen bei Normaldruck durchgeführt.

Bei der Durchführung des erfindungsgemäßen Verfahrens wird bei dem aus jeweils ungefähr 1 Mol der Ausgangskomponenten der Formeln (II), -(III) und (IV) (Reaktionsgemisch (1)) sowie der Formeln (V) und (VI) (Reaktionsgemisch (2)) erhaltenen Gemisches ca. 1 Mol des Oxidationsmittels Wasserstoffperoxid hinzugegeben.

Eine Oxydationslösung läßt sich beispielsweise wie folgt herstellen: Eine Mischung aus 0,01 bis 5,0 g, vorzugsweise 0,05 g bis 3,0 g Katalysator und 20 bis 700 ml, vorzugsweise 50 bis 500 ml Wasser wird tropfenweise und gegebenenfalls unter Kühlung mit 1,8 bis 4,5 Mol, vorzugsweise 1,9 bis 3,5 Wasserstoffperoxid versetzt.

Nach Ablauf der Reaktion wird das entstandene Produkt in üblicher Weise aufgearbeitet, das heißt z.B. der entstandene Niederschlag wird abgesaugt, gewaschen und getrocknet.

Verbindungen der Formel (I) fallen in der Regel in fester Form an und können durch Umkristallisation gereinigt werden. Zur Charakterisierung dient der Schmelzpunkt.

Die nach dem erfindungsgemäßen Verfahren hergestellten 2-Alkylsulfonylalkylen-pyrimidine dienen als Zwischenprodukte für hochwirksame Insektizide (vgl. DE-OS 2 838 359, DE-OS 2 928 185 und DE-OS 3 211 035).

Herstellungsbeispiel

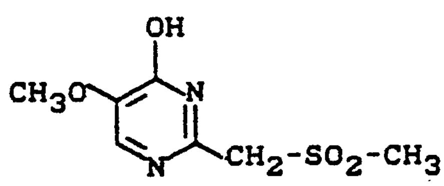

Zu einer Lösung von 0,3 kg (5,5 Mol) Natriummethylat in 7,3 kg (228 Mol) Methanol werden unter Stickstoff bei 0 ° -5 ° C mit 8,4 kg (112 Mol) Chloracetonitril und dann mit 6,5 kg (123 Mol) Ammoniumchlorid versetzt. Bei gleicher Temperatur wird mit 24 kg 25 %iger methanolischer Natriummethylatlösung und mit 5,6 kg (117 Mol) Methylmercaptan versetzt. Diese Reaktionsmischung wird bei 20 ° C zu einem Reaktionsgemisch aus 21 kg (388 Mol) Natriummethylat, 22 kg (688 Mol) Methanol, 24 kg Xylol sowie 10,4 kg (100 Mol) Methoxyessigsäuremethylester und 7,2 kg (120 Mol) Methylformiat, welches vorher 3 Stunden bei 20 ° C gerührt wird, gegeben. Das entstehende Reaktionsgemisch wird 2 Stunden bei 20° C nachgerührt und mit 30 kg Wasser versetzt. Dann wird mit konzentrierter Salzsäure auf pH 4,5 -5,0 gestellt. Das Reaktionsgemisch wird dann im Vakuum eingeengt (ca. 5 l) und mit 0,2 kg Ammoniummolybdat versetzt. Anschließend wird bei einer Temperatur von 40 ° bis 45 ° C 34 kg (300 Mol) 30 %iges Wasserstoffperoxid zugegeben, 30 Minuten bei 40° bis 45 ° C nachgerührt und 30 Minuten bei 5 ° C nachgerührt. Das ausgefallene Produkt wird abgefiltert, mit kaltem Wasser nachgewaschen und getrocknet. Man erhält auf diese Weise 17,7 kg (66 % der Theorie) 4-Hydroxy-5-methoxy-2-methylsulfonylmethyl-pyrimidin mit dem Schmelzpunkt 230° C.

## Ansprüche

1. Verfahren zur Herstellung von 2-Alkylsulfonylalkylenpyrimidinen der Formel (I)

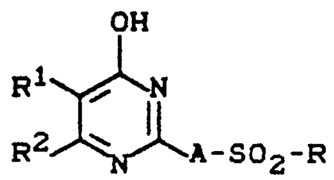

(I)

in welcher

R für geradkettiges oder verzweigtes Alkyl steht,

$R^1$ für Wasserstoff oder für gegebenenfalls substituierte Reste aus der Reihe Alkyl, Alkoxy oder Alkylsulfonyl steht,

$R^2$ für Wasserstoff oder gegebenenfalls substituiertes Alkyl steht und

A für geradkettiges oder verzweigtes Alkylen steht,

dadurch gekennzeichnet, daß man zum Erhalt des Reaktionsgemisches (1) Chloralkylennitrile der Formel (II)

Cl-A-CN (II)

in welcher

A die oben angegebene Bedeutung hat,

mit Ammoniumhalogeniden der Formel (III)

$NH_4X$ (III)

in welcher

X für Halogen steht,

und Alkylmercaptanen der Formel (IV)

RSH (IV)

in welcher

R die oben angegebene Bedeutung hat,

in Gegenwart von Säureakzeptoren und in Gegenwart von Verdünnungsmitteln, bei Temperaturen zwischen -20° C und +60° C versetzt; weiterhin zum Erhalt des Reaktionsgemisches (2) Essigsäureester der Formel (V)

$R^1$-$CH_2$-$COOR^3$ (V)

in welcher

$R^1$ die oben angegebene Bedeutung hat und

$R^3$ für Alkyl mit 1-4 C-Atomen steht,

mit Estern der Formel (VI)

$R^2$-$COOR^4$ (VI)

in welcher

$R^2$ die oben angegebene Bedeutung besitzt und

$R^4$ für Alkyl mit 1-4 C-Atomen steht,

in Gegenwart von Alkoholat oder einer alkoholischen Alkoholatlösung und in Gegenwart eines inerten Verdünnungsmittels bei einer Temperatur von ca. 0° C bis ca. 40° C versetzt, anschließend bei einer Temperatur von ca. 0° C bis ca. 40° C das Reaktionsgemisch (1) zum Reaktionsgemisch (2) gibt, nach Beendigung der Reaktion den entstandenen Alkohol entfernt, den Rückstand gegebenenfalls mit Wasser und einer Mineralsäure versetzt, gegebenenfalls einengt und ohne weitere Reinigung und/oder Isolierung in Gegenwart katalytischer Mengen eines Vanadium-, Wolfram-oder Molybdänsalzes in Gegenwart von Wasser als Verdünnungsmittel mit Wasserstoffperoxid bei Temperaturen zwischen ca. -20° C und +100° C umsetzt.

2. Verfahren gemäß Anspruch 1) zum Erhalt von Verbindungen der Formel

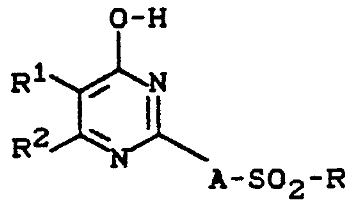

(I)

in welcher

R für geradkettiges oder verzweigtes Alkyl mit 1 bis 6 Kohlenstoffatomen, insbesondere 1 bis 4 Kohlenstoffatomen, steht,

$R^1$ für Wasserstoff oder für Alkyl, Alkoxy oder Alkylsulfonyl, jeweils mit 1 bis 6 Kohlenstoffatomen, insbesondere 1 bis 4 Kohlenstoffatomen, die gegebenenfalls durch Halogen, $C_1$-$C_2$-Alkoxy oder $C_1$-$C_2$-Alkylsulfonyl substituiert sein können, steht,

$R^2$ für Wasserstoff oder Alkyl mit 1 bis 6 Kohlenstoffatomen, insbesondere 1 bis 4 Kohlenstoffatomen, das gegebenenfalls durch Halogen, insbesondere Chlor oder Fluor, $C_1$-$C_2$-Alkoxy oder $C_1$-$C_2$-Alkylsulfonyl substituiert sein kann, steht und

A für geradkettiges oder verzweigtes Alkylen mit 1 bis 6 Kohlenstoffatomen, insbesondere 1 bis 4 Kohlenstoffatomen, steht.

3. Verfahren gemäß Anspruch 1 zum Erhalt der Verbindung der Formel

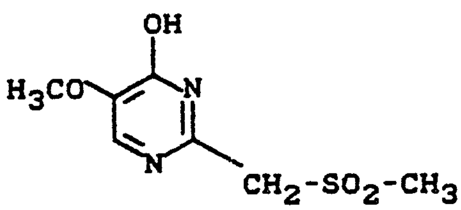

4. Verfahren gemäß Anspruch 1, dadurch gekennzeichnet, daß man zum Erhalt von Reaktionsgemisch (1) Chloracetonitril mit Ammoniumchlorid und Methylmercaptan in Gegenwart von Natriummethylat und in Gegenwart von Methanol umsetzt, zum Erhalt von Reaktionsgemisch (2), Methoxyessigsäureester mit Ameisensäureester in Gegenwart einer methanolischen Methylatlösung und in Gegenwart eines aromatischen Kohlenwasserstoffs umsetzt, anschließend Reaktionsgemisch (1) zum Reaktionsgemisch (2) gibt, mit Wasser versetzt, mit Mineralsäure auf pH 4,5-5,0 stellt, anschließend einengt und mit Wasserstoffperoxid in Gegenwart eines Molybdates umsetzt.